# EUROPEAN PATENT APPLICATION

(11) **EP 4 817 515 A1**
(43) Date of publication of application: **07.10.2026**
(21) Application number: 25168504.6
(22) Date of filing: 04.04.2025
(51) Int. Cl.: A61B 5/08, A61B 5/083, A61B 5/087

(54) **DETECTION OF EXHALED GASES**

(71) Applicant: Koninklijke Philips N.V., 5656 AE Eindhoven (NL)
(72) Inventor: KOOIJMAN, Gerben, Eindhoven (NL); BALTAZAR DOS SANTOS, Nuno Filipe, Eindhoven (NL); VAN LIESHOUT, Ron Martinus Laurentius, Eindhoven (NL)
(74) Representative: Philips Intellectual Property & Standards

(57) **Abstract**

An analysis system is provided for analyzing the breath of a subject. A gas sensor is used to detect a target gas in inhaled and exhaled air of a subject. The timing of breathing inhalation and exhalation phases is determined, and a target gas concentration is obtained which is indicative of a subject-induced generation of the target gas. It is or example derived from fluctuations in the gas sensor output signal between the (i) exhalation phase or a portion thereof, and (ii) the inhalation phase or a portion thereof. This analysis system enables the breathing inhalation and exhalation phases to be resolved so that a target gas measurement can be controlled accordingly. In this way, the analysis system can distinguish between compounds or compound concentrations already present in the ambient environment and compounds or compound concentrations produced by the user.

## Description

### FIELD OF THE INVENTION

This invention relates to the detection of exhaled gases.

### BACKGROUND OF THE INVENTION

Measuring gas compounds in breath can be valuable for assessment of a person's systemic health. Thus, it is known to use gas sensors to monitor the composition of the exhalation of a subject.

Certain adverse health conditions may be detected from the presence of specific gas compounds, or from elevated or lowered levels of specific gas compounds.

A common issue when measuring gas compounds in exhaled breath (or in the oral cavity) is that certain compounds of interest may also be present in the ambient air at a certain concentration level. Furthermore, a gas sensor can suffer from detrimental cross-sensitivity effects, i.e. in addition to exposing a sensor to a gas compound of interest, exposing a sensor to a gas compound that is not the gas compound of interest, can cause an unwanted effect: This cross sensitivity may result in a positive response, a negative response or inhibition to the target gas compound of interest. This is especially a common issue with sensors measuring volatile organic compounds (VOCs).

It is known, for example from US 2016/278664, to integrate breath gas compound sensing in an oral care device, such as a smart toothbrush. This enables systemic health monitoring that is seamlessly integrated in a person's oral care routine. This makes it non-invasive and provides the benefit of generating data points frequently (typically twice per day) and consistently over time, enabling continuous tracking and comprehensive longitudinal analysis.

However, analysis of gas compounds in exhaled breath gives uncertain results because the concentrations present in the exhaled breath will depend on the concentrations present in the inhaled breath. For accurate assessment, ambient gas compounds that are inhaled as well as exhaled by the user should be discerned from gas compounds produced and exhaled by the user or in the oral cavity. This is challenging, particularly, when sensors are to be integrated into an oral care device.

### SUMMARY OF THE INVENTION

The invention is defined by the claims.

According to examples in accordance with an aspect of the invention, there is provided an analysis system for analyzing the breath of a subject, comprising:
a sensor system comprising at least a gas sensor for detecting a target gas that generates an output signal indicating a target gas concentration, wherein the gas sensor is for analyzing inhaled air and exhaled air of the subject; and
a processor for processing an output signal or signals of the sensor system, wherein the processor is configured to:
   determine the timing of breathing inhalation and exhalation phases; and
   derive a target gas concentration which is indicative of a subject-induced generation of the target gas from fluctuations in the gas sensor output signal between the (i) exhalation phase or a portion thereof, and (ii) the inhalation phase or a portion thereof or an ambient target gas concentration level.

This analysis system enables the breathing inhalation and exhalation phases to be resolved so that the target gas measurement can be controlled accordingly. **In** this way, the analysis system can distinguish between compounds or compound concentrations already present in the ambient environment and compounds or compound concentrations produced by the user. Thus, a measure of the subject-induced generation of the target gas can be made.

The ambient gas concentration may be obtained from the gas sensor reading during inhalation, or it may be a known and constant concentration level if the target gas is a basic component of ambient air.

The ambient gas concentration may also be measured at a different moment in time, thus not during inhalation (when the device is placed in the mouth). For instance, an ambient background measurement or sample collection may be performed just before the oral care device is placed in the mouth,

However, in a preferred implementation, the target gas concentration is derived from fluctuations in the gas sensor output signal between the (i) exhalation phase or a portion thereof, and (ii) the inhalation phase. Thus, the timing of breathing inhalation and exhalation phases is used to enable the exhalation concentration and the inhalation concentration to be determined, and all required measurements are taken during the normal use of the device which incorporates the analysis system, without requiring a specially timed, or separate, measurement.

When a target gas is inhaled as well as exhaled, a difference in concentration can be used to derive a measure of the subject-induced generation of the target gas.

The gas detector may be used to detect inhaled and exhaled air in real time, or air samples may be taken for target gas detection at a different location and/or time.

The processor may for example be configured to determine an ambient concentration of the target gas from the output signal of the gas sensor in respect of the inhalation phase and to determine an exhaled concentration of the target gas from the output signal of the gas sensor in respect of the exhalation phase or a portion thereof.

The processor is for example configured to identify the timing of the breathing inhalation and exhalation phases from fluctuations in the gas sensor output signal. **In** this way, the gas sensor used to detect the target gas may itself be used to distinguish between inhalation and exhalation phases. This means that an additional sensor for detecting the breathing phase is not required.

**In** examples, the sensor system may further comprise a (separate) breathing phase sensor for identifying the timing of the breathing inhalation and exhalation phases and the processor is configured to identify the timing of the breathing inhalation and exhalation phases from an output of the breathing phase sensor. **In** this case, a separate breathing sensor is used, additionally to the gas sensor. The breathing phase sensor for example comprises an O₂ concentration sensor, or a CO₂ concentration sensor, a temperature sensor, a relative humidity sensor, or a flow sensor. Thus, a choice of known sensors may be used for breathing phase detection.

The breathing phase sensor may for example comprise an inert gas concentration sensor and the processor is configured to derive a gas temperature from the inert gas concentration. This provides one possible way to measure temperature indirectly, for detecting inhalation and exhalation.

The processor may be configured to determine a plurality of sub-phases of the exhalation phase. **In** this way, target gas analysis is possible for different exhalation phases.

A more granular detection of the exhalation phase (and optionally also the inhalation phase) for example enables the system to discriminate between gas compounds in exhalation, the gas samples will originate from the mouth or gastrointestinal tract, whereas from later exhalation, the gas samples will originate from the lungs. **In** this way, the detection may be directed to target gases that originate from oral health conditions or from lung health conditions.

The target gas may for example comprise a volatile organic compound (VOC ) such as acetone, ketone, heptane, and ammonia. By discretizing the different exhalation phases, it becomes possible to identify the source of the VOCs. Sulphur based compounds are also of interest because they can be present in exhaled breath but also in the oral cavity and in the environment as well.

The processor for example comprises a gated amplifier that is gated using the timing of breathing inhalation and exhalation phases to provide amplification during the exhalation phase.

The analysis system may further comprise a gas sampling system, wherein the gas sampling system is controlled using the timing of breathing inhalation and exhalation phases to provide sampling during the exhalation phase or sampling during the inhalation and exhalation phases.

When controlling gas sampling based on the inhalation/exhalation phase, a sample may be taken only for the exhalation phase. The exhalation gas concentration is measured from this sample, whereas the inhalation or ambient concentration may then simply be an assumed known value.

The gas sensor may thus be used for analyzing sampled air, and hence it may be performed at a different point in time, such as after an oral care session, and at a different location, such as at a base station of a device incorporating the analysis system.

The invention also provides a toothbrush comprising:
a brushing head;
a handle; and
an analysis system as described above.

The gas sensor of the sensor system is for example located on the handle, or a gas sampling system of the analysis system is located on the handle. In this way, the gas may for example be sensed or sampled from below the nose while the user is brushing their teeth.

In one example, the toothbrush may further comprise a location system for determining the location of the toothbrush head within the mouth during brushing, wherein the processor is configured to control gas sampling, or perform analysis of the gas sensor output signal, when the location system detects brushing of a particular mouth region.

Brushing of this particular mouth region is for example indicative of the gas sensor, or sampling system, being at a suitable location in the flow of exhaled nasal breath.

In another example, the processor may be configured to determine, from the output of the gas sensor, when the gas sensor is located along the path of an exhaled airflow. In this way, the detection of a target gas itself is indicative that the gas sensor is located in the flow of exhaled breath.

The invention also provides a computer program comprising computer program code means that is adapted, when said program is run on a processor of an analysis system for analyzing the breath of a subject, the method comprising:
receiving an output signal from a sensor system comprising at least a gas sensor for detecting a target gas that generates an output signal indicating a target gas concentration, wherein the gas sensor is for analyzing inhaled air and exhaled air of the subject; and
processing the output signal of the sensor system to:
   determine the timing of breathing inhalation and exhalation phases; and
   derive a target gas concentration which is indicative of a subject-induced generation of the target gas from fluctuations in the gas sensor output signal between the (i) exhalation phase or a portion thereof, and (ii) the inhalation phase or a portion thereof or an ambient gas concentration level.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a powered toothbrush;
Fig. 2 shows a fluctuating target gas concentration measurement;
Fig. 3 shows a first example of components of the analysis system;
Fig. 4 shows the flows in the vicinity of a flow sensor during inhalation and exhalation;
Fig. 5 shows an example of processing for the examples using an additional breathing phase sensor;
Fig. 6 shows a CO₂ gas sensor output signal over time and is used to explain how it may be used to determine when the gas sensor is directly in the exhalation airstream;
Fig. 7 shows an alternative design in which a breathing phase sensor is used to control a gas sampling system; and
Fig. 8 shows computer-implemented analysis method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an analysis system for analyzing the breath of a subject. A gas sensor is used to detect a target gas in inhaled and exhaled air of a subject. The timing of breathing inhalation and exhalation phases is determined, and a target gas concentration is obtained which is indicative of a subject-induced generation of the target gas. It is for example derived from fluctuations in the gas sensor output signal between the (i) exhalation phase or a portion thereof, and (ii) the inhalation phase or a portion thereof. This analysis system enables the breathing inhalation and exhalation phases to be resolved so that a target gas measurement can be controlled accordingly. In this way, the analysis system can distinguish between compounds or compound concentrations already present in the ambient environment and compounds or compound concentrations produced by the user.

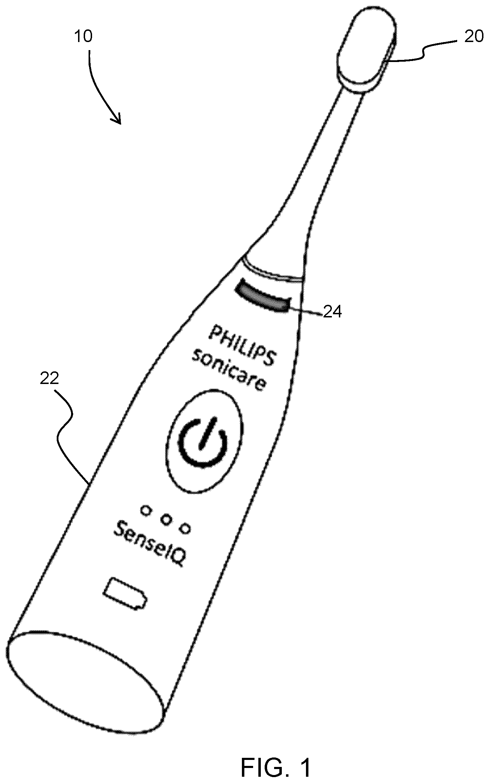

Fig. 1 shows a powered toothbrush 10 comprising a brushing head 20 and a handle 22.

The toothbrush incorporates an analysis system which includes a sensor system having at least a gas sensor for detecting a target gas.

In Fig. 1, the gas sensor is behind a gas sensing window 24 on a front face of the handle. The gas sensing window 24 is intended to be located in the path of exhaled nasal air at certain points in time during a brushing routine of the user.

The gas sensor generates an output signal indicating a target gas concentration. In Fig. 1, the gas sensor is thus in the path of inhaled air and exhaled air of the user during brushing, but more generally the gas sensor is for analyzing inhaled air and exhaled air. The inhaled and exhaled air may for example have been previously sampled by a gas sampling system which is then located on the handle, for example behind a gas sampling window.

A processor is used to process an output signal or signals of the sensor system to determine the timing of breathing inhalation and exhalation phases and derive a target gas concentration which is indicative of a subject-induced generation of the target gas. The subject-induced target gas concentration provides information on a health condition of the user.

For the basic implementation of Fig. 1 with a gas sensor at the toothbrush handle, if the user breathes through the nose while using the toothbrush, the sensor is exposed to ambient air during inhalation and to air exhaled by the user during exhalation.

**In** an implementation with fewest components. distinguishing the gas compound concentrations in the ambient air and in the exhaled breath (hence performing breathing phase detection) may be based on the gas sensor signal itself. Thus, the gas sensor may also function as a breathing phase sensor. For example, when for a certain compound it can be assumed that the concentration in exhaled breath is higher than the ambient concentration, peaks and valleys in the signal can be associated with exhalation and ambient surroundings, respectively.

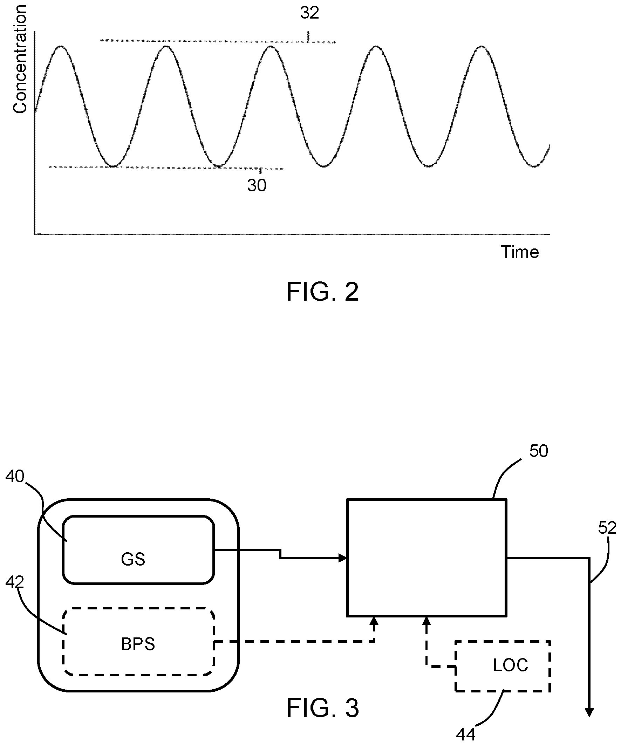

Fig. 2 shows a fluctuating target gas concentration measurement. The peaks correspond to exhalation times and the valleys correspond to inhalation times. Line 30 represents the ambient concentration level and line 32 represents the concentration level in exhaled breath. The difference between the levels 30, 32 is the target gas concentration which is indicative of a subject-induced generation of the target gas.

For semiconductor-based gas sensors, temperature affects the sensor output. This means that the sensor output for an inert gas (i.e., a gas compound for which concentration in inhaled and exhaled air will be the same) can in fact be used as a temperature sensor. Thus, the gas sensor itself may function as a temperature sensor, which can then be used for breathing phase detection.

For this purpose, the inert gas sensor will be temperature-calibrated for the known inert gas concentration in ambient air, such that the concentration measurement has a known correlation with temperature. The inert gas may be a chemically inert gas of the periodic table (Helium, Argon, Neon, Krypton, Xenon or Radon) but it may be other gas compounds that will not be affected or changed by the user, i.e. a 'physiologically inert gas', such as nitrogen (N₂),

**In** order to determine the inert gas concentration, so that the gas measurement can be correlated to temperature, a local calibration may be carried out. The concentration measurement is used to detect meso or micro time level temperature changes.

When employing a semiconductor sensor array, with sensitivity for one or more target gases as well as for an inert gas, the breathing phases can then be detected based on the fluctuation in the inert gas sensor output (due to temperature fluctuation), and the target gas sensor output can be processed accordingly.

Fig. 3 shows the components of the analysis system. The sensor system comprises a gas sensor 40, and the output of the gas sensor is provided to a processor 50, which derives subject-induced gas concentration 52.

The gas sensor for example comprises a semiconductor (e.g. MOS) electrochemical sensor that detects electrical (e.g. resistance) changes when exposed to specific target compounds.

Certain sensors for measuring specific gas compounds or types of gas compound may have a relatively slow response time. This is typically the case for Volatile Organic Compound (VOC) sensors. They may also suffer from noise. Furthermore, it may not always be known how the concentration level in exhaled air qualitatively compares to that in the ambient air: In other words, no assumption can be made on whether the concentration in exhaled breath is higher or lower than ambient. In such cases the basic embodiment above may be inadequate.

For this reason, an option is to provide an additional sensor 42 (as part of the sensor system) to detect the breathing phases. The inhalation and exhalation phases are determined from the signal of the additional sensor 42. The ambient and exhalation gas compound concentrations are for example calculated from averaging the gas sensor signal over these respective phases.

In one set of examples, the sensor 42 may comprise a sensor that measures O₂ concentration, CO₂ concentration, (relative) humidity, or temperature. As mentioned above, the gas sensor itself may be used to measure temperature, and if the target gas is O₂ or CO₂ then again the gas sensor itself may function as the breathing phase sensor. A separate temperature sensor may instead be used, and if the target gas is a VOC for example, then an additional O₂ or CO₂ sensor may be used as the breathing phase sensor.

The values of these possible parameters clearly vary between ambient air and exhaled breath. More specifically, when breathing normally and without considering extreme altitudes, humans inhale air that contains 21% of O₂ and a negligible amount of CO₂ (0.04%), and exhale approximately 16% O₂ and approximately 5% CO₂.

The additional sensor 42 is chosen to have fast enough response times to be able to follow a breathing cycle. The signal of the additional sensor may be used in the processing of the primary gas sensor signal.

Another option for the breathing phase sensor 42 is a flow sensor, which may again be mounted on the toothbrush handle. Such a sensor also typically has a fast enough response to resolve the breathing cycle.

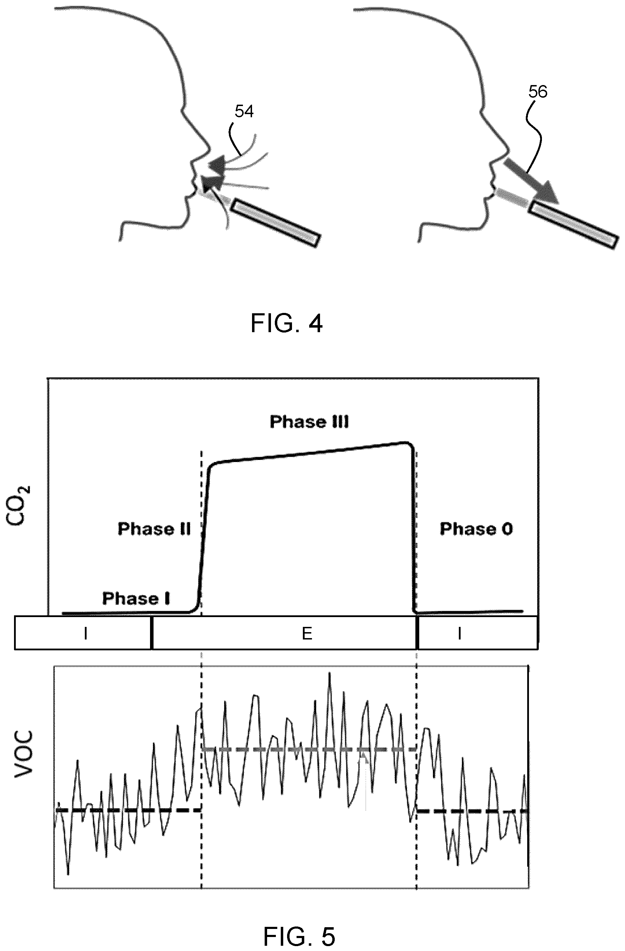

Fig. 4 shows the flows in the vicinity of the flow sensor during inhalation (left image) and exhalation (right image). With the user breathing through the nose, air will be drawn from all directions during inhalation as shown by arrows 54, which results in virtually zero flow measured by the sensor. However, during exhalation, the expelled air is directed in the form of a jet 56 towards the toothbrush handle, which results in flow measured by the sensor.

Fig. 5 shows an example of processing for the examples using an additional sensor 42, illustrated for one breathing cycle. The additional sensor is in this example shown as a CO₂ sensor, and the top plot shows the measured CO₂ concentration over time. The bottom plot shows the gas sensor output for a VOC sensor.

The breathing phases (Inhalation, I and exhalation, E) are determined from the CO₂ sensor signal. The concentrations of the gas compound of interest (e.g. a specific VOC) in the exhaled air and in the ambient environment are then obtained by averaging the gas sensor signal over the determined expiration phase and inspiration phase, as shown by the dotted lines. The expiratory pause may also be determined, and the concentrations of the gas compound of interest may also be determined during the expiratory pause, to give additional information for interpretation.

More advanced processing may be performed based on lock-in amplification for example where the additional sensor signal is used as the reference signal. The processor may then comprise a gated amplifier that is gated using the timing of breathing inhalation and exhalation phases to provide amplification during the exhalation phase.

Returning to Fig. 3, there is also shown schematically a further optional sensing feature in the form of a location system 44 for determining the location of the toothbrush head within the mouth during brushing. This may also be considered to be part of the overall sensor system.

Toothbrush location sensing is already a feature of existing toothbrushes, using an IMU sensor, The location sensing estimates the positioning of the brush head in the user's mouth. From this, the positioning of the handle with respect to the user's face can be estimated.

When brushing molars, the handle is typically closer to the nose (more or less positioned under the nose), whereas when brushing the front teeth, the handle will be further away from the user's nose. This information can be used to further improve the estimation of exhaled gas versus ambient gas compound concentration. For instance, the target gas measurement is only processed over the time intervals during which the handle with the gas sensor is close to the nose.

Alternatively, or additionally, it may be deduced from the gas sensor signal itself, whether it is positioned in the exhalation airstream. For instance, when using a CO₂ sensor, small fluctuations over time in the sensor signal can be expected when the toothbrush handle and its sensor are not close to the exhalation airstream. When the handle and sensor are positioned under the nose and are directly in the airstream, a maximum fluctuation in signal, correlating to the inhalation and exhalation phases, can be expected. For optimum accuracy, the target gas sampling or measurement may be processed only for the parts where the detected (fluctuation in) CO₂ level is above a certain threshold.

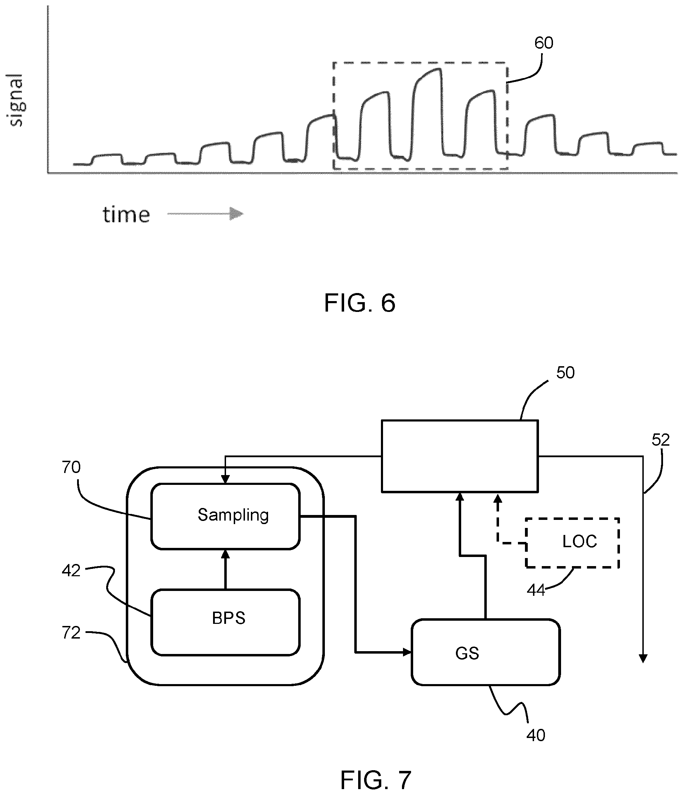

Fig. 6 shows a CO₂ gas sensor output signal over time. The signal amplitude varies in dependence on the orientation and position of the toothbrush. The processing of the gas sensor signal may be used to determine when the gas sensor is directly in the exhalation airstream. When the toothbrush handle with its sensor is moving over time into the exhalation airstream, an increase in CO₂ signal fluctuation, timed with the inhalation and exhalation phases, can be expected. When it moves out of the airstream, the signal amplitude fluctuation will become smaller again. For optimal accuracy in sampling of the target gas or processing of the target gas measurement, only portions where the CO₂ signal fluctuation is above a certain threshold may be taken into account as illustrated by the region 60.

Fig. 7 shows an alternative design in which a breathing phase sensor 42 (as discussed with reference to Fig. 3) may be used to control a gas sampling system 70. Thus, the components at the toothbrush handle (indicated by box 72) include the breath detection and sampling system, but the gas sensing may be at a different location. For example, the stored gas samples may be delivered to the gas sensor after the toothbrushing session. The gas sampling system 70 is controlled using the timing of breathing inhalation and exhalation phases to provide sampling during the exhalation phase or sampling during the inhalation and exhalation phases. For instance, gas during inhalation is sampled and collected separately from gas during exhalation.

A most basic implementation of the gas sampling system would be a single chamber that is opened using a controlled valve just once during a detected exhalation. Repeatedly opening a sampling chamber during exhalation, in order to accumulate a sample, would also be an option, in particular when an absorbing material is used in the sampling chamber.

There may be separate chambers for gas accumulation during the inhalation and exhalation phases. Each chamber for example has an absorber material to collect the target volatiles or target gas.

Another option is to purge a single gas chamber after a measurement is completed, so that a new sample can be taken and measured. The chamber or chambers, when closed, are sealed to prevent contamination.

After purging, the baseline measurement will correspond to ambient air that is trapped inside the chamber once it closes again, waiting for the next sampling phase and measurement.

A single chamber may be used for the measurement of multiple target gases, for example different VOC targets, with the same sensor by using a time division multiplexing approach.

**In** another approach, there may be an array of chambers so that a time sequence of samples is obtained. The sampling may be passive, although additional means (e.g. suction) could also be applied.

User-produced gas compounds in exhaled air can originate from lungs, mouth, and gastrointestinal tract. Especially when measuring exhaled air from the nose on an oral care device handle, mainly gas compounds from the lungs will be measured.

However, the first part of an exhalation phase can still contain some gases from the oral cavity and/or gastrointestinal tract. Hence, for distinction of gas compound (concentration) in the ambient, the lungs as well as mouth and gastrointestinal tract, a further refinement in detected breathing phases may be employed. The processor may for example be configured to determine a plurality of sub-phases of the exhalation phase. Two sub-phases may for example comprise early exhalation and main exhalation. However, three or more sub-phases may be defined.

**In** the examples above, the gas concentration measured during inhalation is used to represent the ambient concentration. If the target gas concentration in the ambient surroundings is known then it does not need to be measured. Alternatively, it may be obtained from a separate gas sensor that is remote from the user's breath.

By way of example, the target gas may comprise one or more of acetone, ketone, heptane or ammonia. Sulphur-based compounds as target gas are also interesting because they can be present in exhaled breath but also in the oral cavity and in the environment as well. These VOCs may also be present to some extent in the ambient environment, of course highly dependent on the actual environment conditions.

By way of example, the presence of certain VOCs in exhaled breath may be associated with risk factors for obstructive pulmonary diseases, as discussed in "Exhaled volatile organic compounds associated with risk factors for obstructive pulmonary diseases: as systematic review" S. Shahbazi Khamas et. al., ERJ Open Res 2023; 9: 00143-2023.

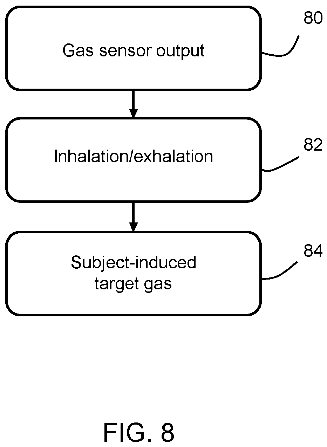

Fig. 8 shows the analysis method that is implemented by a computer program running on the processor 50.

In step 80, an output signal is received from the sensor system (the gas sensor and any optional location or breathing phase sensors). The output signal may be received in real time during a brushing routine, or it may relate to post-processing of samples that were collected during the brushing routine.

In step 82, the timing of breathing inhalation and exhalation phases is determined. This may involve analyzing the real time gas sensor signal or breathing phase signals, or it may involve identifying the collected samples as inhalation samples or exhalation samples.

In step 84, a target gas concentration is derived which is indicative of a subject-induced generation of the target gas.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An analysis system for analyzing the breath of a subject, comprising:
a sensor system comprising at least a gas sensor (40) for detecting a target gas that generates an output signal indicating a target gas concentration, wherein the gas sensor (40) is for analyzing inhaled air and exhaled air of the subject; and
a processor (50) for processing an output signal or signals of the sensor system, wherein the processor is configured to:
determine the timing of breathing inhalation and exhalation phases; and
derive a target gas concentration which is indicative of a subject-induced generation of the target gas from fluctuations in the gas sensor output signal between the (i) exhalation phase or a portion thereof, and (ii) the inhalation phase or a portion thereof or an ambient target gas concentration level.

2. The analysis system of claim 1, wherein the processor (50) is configured to determine an ambient concentration of the target gas from the output signal of the gas sensor in respect of the inhalation phase and to determine an exhaled concentration of the target gas from the output signal of the gas sensor in respect of the exhalation phase or a portion thereof.

3. The analysis system of claim 1 or 2, wherein the processor (50) is configured to identify the timing of the breathing inhalation and exhalation phases from fluctuations in the gas sensor output signal.

4. The analysis system of claim 1 or 2, wherein the sensor system further comprises a breathing phase sensor (42) for identifying the timing of the breathing inhalation and exhalation phases and the processor (50) is configured to identify the timing of the breathing inhalation and exhalation phases from an output of the breathing phase sensor.

5. The analysis system of claim 4, wherein the breathing phase sensor (42) comprises an O₂ concentration sensor, a CO₂ concentration sensor, a temperature sensor, a relative humidity sensor, or a flow sensor.

6. The analysis system of claim 4, wherein the breathing phase sensor (42) comprises an inert gas concentration sensor and the processor (50) is configured to derive a gas temperature from the inert gas concentration.

7. The system of any one of claims 1 to 6, wherein the processor (50) is configured to determine a plurality of sub-phases of the exhalation phase.

8. The analysis system of any one of claims 1 to 7, wherein the processor (50) comprises a gated amplifier that is gated using the timing of breathing inhalation and exhalation phases to provide amplification during the exhalation phase.

9. The analysis system of any one of claims 1 to 8, further comprising a gas sampling system (70), wherein the gas sampling system (70) is controlled using the timing of breathing inhalation and exhalation phases to provide sampling during the exhalation phase or sampling during the inhalation and exhalation phases.

10. A toothbrush (10) comprising:
a brushing head (20);
a handle (22); and
an analysis system as claimed in any one of claims 1 to 9.

11. The toothbrush of claim 10, wherein the gas sensor (40) of the sensor system is located at the handle or a gas sampling system (70) of the analysis system is located at the handle.

12. The toothbrush of claim 11, further comprising a location system (44) for determining the location of the toothbrush head within the mouth during brushing, wherein the processor (50) is configured to control gas sampling, or perform analysis of the gas sensor output signal, when the location system (44) detects brushing of a particular mouth region.

13. The toothbrush of claim 11, wherein the processor (50) is configured to determine, from the output of the gas sensor, when the gas sensor (40) is located along the path of an exhaled airflow.

14. A computer program comprising computer program code means that is adapted, when said program is run on a processor of an analysis system for analyzing the breath of a subject, the method comprising:
receiving an output signal from a sensor system comprising at least a gas sensor for detecting a target gas that generates an output signal indicating a target gas concentration, wherein the gas sensor is for analyzing inhaled air and exhaled air of the subject; and
processing the output signal of the sensor system to:
determine the timing of breathing inhalation and exhalation phases; and
derive a target gas concentration which is indicative of a subject-induced generation of the target gas from fluctuations in the gas sensor output signal between the (i) exhalation phase or a portion thereof, and (ii) the inhalation phase or a portion thereof or an ambient gas concentration level.

15. The computer program of claim 14, wherein the method further comprises determining a plurality of sub-phases of the exhalation phase and optionally also a plurality of sub-phases of the inhalation phase.
